# EUROPEAN PATENT SPECIFICATION

(11) **EP 2 953 668 B1**
(45) Date of publication and mention of the grant of the patent: **13.09.2017**
(21) Application number: 14705596.6
(22) Date of filing: 10.02.2014
(51) Int. Cl.: A61M 5/30, A61M 35/00, A61M 37/00, A61N 5/06

(54) **DEVICE FOR INJECTING A DRUG**
VORRICHTUNG ZU EINSPRITZEN EINER ARZNEI
DISPOSITIF PERMETTANT D'INJECTER UN MÉDICAMENT

(30) Priority: 11.02.2013 EP 13154807
(43) Date of publication of application: 16.12.2015
(73) Proprietor: Nederlandse Organisatie voor toegepast- natuurwetenschappelijk onderzoek TNO, 2595 DA 's-Gravenhage (NL)
(72) Inventor: PERINCHERY, Sandeep Menon, NL-2595 DA's Gravenhage (NL); SCHOO, Harmannus Franciscus Maria, NL-2595 DA's Gravenhage (NL); UNNIKRISHNAN, Sandeep, NL-2595 DA's Gravenhage (NL)
(74) Representative: V.O.
(86) International application number: PCT/NL2014/050078
(87) International publication number: WO 2014/123421

(56) References cited:
- EP-A1- 2 514 477
- WO-A1-94/28874
- WO-A1-2012/146652
- WO-A2-2012/035429
- US-A1- 2011 230 826
- MI-AE PARK ET AL: "Er:YAG laser pulse for small-dose splashback-free microjet transdermal drug delivery", OPTICS LETTERS, OSA, OPTICAL SOCIETY OF AMERICA, WASHINGTON, DC, US, vol. 37, no. 18, 15 September 2012 (2012-09-15), pages 3894-3896, XP001578534, ISSN: 0146-9592 [retrieved on 2012-09-14] cited in the application

## Description

### FIELD OF INVENTION

The invention relates to a Device for injecting a drug.

### BACKGROUND

In the art, a variety of methods exist as alternatives for drug delivery via needle-based and also nozzle-based (i.e. needle-free or jet) injection. Commercially available liquid jet injectors use compressed gas or spring to create high pressure jets. The current techniques are efficient; however, there are some limitations.
1) Drug volume resolution is restricted (above ∼200 nl).
2) Depending on nozzle size, a delivered drug volume is difficult to control over a large volume range;
3) Nozzle type of drug delivery experiences sanitation problems related clogging.
4) The techniques utilize complex mechanical arrangements.
5) Jetted drug delivery often requires cumbersome gas cartridges.
6) Heat, friction and / high pressure effects may thermalize the active compounds of the drug when supplied via a nozzle.

In "Er:YAG laser pulse for small-dose splashback-free microjet transdermal drug delivery" Mi-ae Park et al. Optics letters / Vol. 37, No. 18 / September 15, 2012, a method is described wherein a fluid jet is formed by a membrane that acts in response to a vapour bubble by laser pulsing. The fluid jet ejects from a nozzle, at sufficient velocity needed to penetrate the skin. The disclosure describes that this type of delivery eliminates needle waste and provides a favourable alternative for needle-phobic patients. However, this type of jet forming is inadequate for a variety of drugs, inter alia since a nozzle is used for providing a fluid jet. The current invention aims at providing a deliverydevice, wherein the advantages of micro dose jet delivery can be attained wherein a wider range of applications is possible.

### SUMMARY OF THE INVENTION

To this end, it is proposed to provide a device for drug supply, the device comprising:
- a holder suitable for holding a carrier comprising a drug layer;
- a transmission part for transmitting a light beam from a light system, via the transmission part to the carrier;
- a placement provision; arranged for distancing the carrier from the skin or tissue; and
- a control mechanism arranged to
   ∘ moving the light beam and/or the carrier relative to each other, in order to target a virginal area of the carrier for the light beam;
   ∘ impinging the light beam via the transmission part on the carrier; in such a way that the carrier is activated to eject a distinct quantity of drug transferred (50) in normal direction from the carrier from the drug layer to the skin or tissue; and
   ∘ repeating the steps of moving and impinging in a computer-controlled way, in order to supply a predetermined quantity of drug to the skin or tissue. Accordingly, a drug delivery device is provided that facilitates needle free injection based on laser induced forward transfer technique. This could enable the jetting material irrespective of fluid or solid using the same device without a nozzle head. In principle, this could be utilized to deliver drugs, insulin, proteins, antigens, cells etcetera on or into human skin or other parts, in quantities ranging from picoliter to milliliter volumes. In particular, the device and disclosed techniques enable drug supply in quantities with smaller resolution than the existing techniques. This could eventually allow injection with reduced pain.

### BRIEF DESCRIPTION OF THE FIGURES

Figure 1 illustrates schematically a drug delivery device according to an embodiment.
Figure 2 shows a schematic operation of the device of figure 1.
Figure 3 schematically provides elevational and side views of a pre-patterned donor cartridge.
Figure 4 shows side views of a carrier that may be used to direct the drug substance in a prescribed direction.
Figure 5 shows a cartridge system functioning as a carrier transport mechanism.
Figure 6 shows an example of a donor feed system
Figure 7 shows an example of a structured donor film.

### DETAILED DESCRIPTION

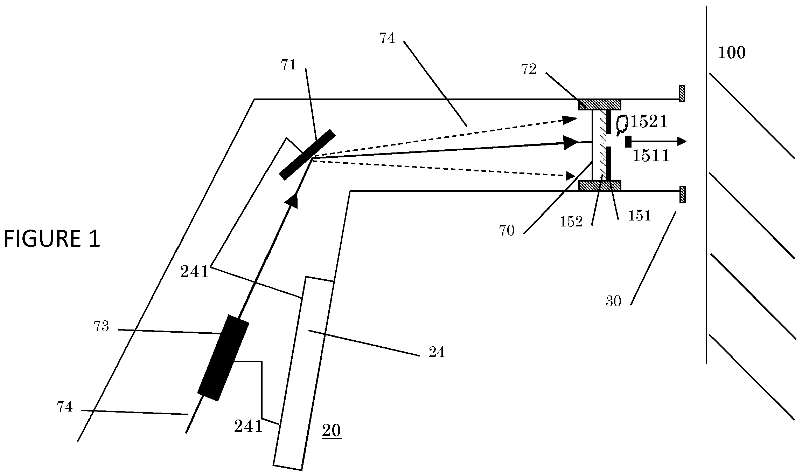

Figure 1 illustrates schematically a drug delivery device implementing a method according to an embodiment. A light source is provided (not shown: a laser, LED or suitable alternative), to generate enough energy to generate donor jet via a laser induced forward transfer (LIFT) effect from a drug layer 151. Preferable a laser source is provided (fiber or microchip based) which works around 350 nm with nano second pulsing.

The laser spot used for LIFT could have a diameter ranging from 5 microns-5 mm. The wavelength of the pulsed laser could be in the range of 200 nm-1200 nm (and could be of nanosecond or picosecond or femtosecond type). The fluence of the laser could be in the range of 10 mJ/cm2 - 10 J/cm2.

The light spot is aimed on a transparent carrier substrate 70, for example, a transparent carrier such as glass, film or tape; a quartz glass for a 248 nm KrF excimer and PET film or Soda Lime Glass for a 355nm Nd:YAG laser. On the substrate 70 a thin layer of a dynamic release layer is applied. This may be a thin layer (80 to 200 nm) of Triazene polymer that ejects, under the influence of the light beam 74 a propelling gas for transfer of the drug from the drug layer. Different dynamic release layer materials could potentially be used such as polymers with metallic nanoparticles, or a combination of triazene polymer layer and thin metal layer. Adjacent this layer 152, a drug layer 151 is provided with an active substance. In the example, the dynamic release layer 152 is formed by a triazene layer of about 100 nm thickness which functions as a sacrificial dynamic release layer (DRL), and comprises a polymer that, when photoactivated decomposes into nitrogen and other organic volatile gases 1521. A typical peak absorption is found at 290-330 nm and the ablation threshold: 22-32 mJ/cm2 at 308 -248 nm is quite low so that the layer 151 is neither thermally loaded or optically degraded and remains intact after transfer, in such a way that the active drug substance 1511 is preserved. For example, the laser beam may be restricted in timing and energy. Accordingly a desired property of the drug substance can be retained during transfer by impinging the laser beam on the dynamic release layer 152 adjacent to the drug layer 151; in order to supply a predetermined quantity of drug to the skin or tissue 100.

To illustrate the general applicability of the drug delivery method solids, fluids or even highly viscous substances may be transferred, for example, with a viscosity of 1 - 180 Pa.s. The layer 151 may be provided as homogenous layer of 20-30 micron, in particular, 25 micron thick provided on the dynamic release layer 152. The thickness is controlled to be around 25 um or 50 um but could be of any thickness. The donor drugs may be provided in a cartridge 72 that is held at a distance of about 1-5 millimeter away from the skin or tissue by spacers 30 that function as a placement provision arranged for distancing the carrier from the skin or tissue.

The device further comprises a transmission part 71 that transmits the laser beam 74 to a suitable spot on the substrate 70. The transmission part, in the example, may comprise a beam deflector 71 that is controlled to target a virginal area by deflection of said beam 74 over the donor film. The laser beam is (optionally) scanned over the substrate thereby efficiently using all area containing the drug substance. The scanning may be done using a MEMS mirror. If the substrate width is almost the same as the laser spot size, then beam scanning may not be necessary.

Scanning the laser beam 74 can typically be arranged by a MEMS scanning xyz stage for scanning the laser beam over the donor cartridge 72. While a transmission part 71 may be formed by a single aperture, typically, the transmission part may comprise a variety of optical elements, for instance, a shutter, beam expander 73 and/or focusing elements to change the spot size of the beam at the location where it impinges on the carrier 70. The transmission part may be a fast beam modulator 71 (galvano mirror, polygon mirror, acousto-optic or electro-optic modulator etc.) may provide a scanning movement of the laser beam in a first direction. The modulator may be controlled in a feed forward process. Optionally, a main beam is split into about 2-20 sub beams.

The device 20 can be designed as a handheld device, for example, including a pistol grip (not shown), and the light source included in the holder. The device 20 further comprises a control mechanism, for example, a microprocessor controller 24 arranged on a wiring board. The controller is connected via connection lines 241 to various items that function for moving the light beam and/or the carrier relative to each other, in order to target a virginal area of the carrier 70 for the light beam. Such a virginal area may be described as an area that is not targeted previously, or at least prepared in a way that a dynamic release layer 152 can function to eject, under the influence of the light beam 74, a propelling gas 1521 for transfer of the drug from the drug layer 151. The controller 24 further functions to have the light beam imping, via the transmission part 71 on the carrier 70; in such a way that the carrier is activated, in the example via the release layer 152, to eject a distinct quantity of drug transferred (50), by light induced propulsion, in normal direction from the carrier from the drug layer to the skin or tissue. The controller 24 may function as a dosage counter that can be preprogrammed or even hardwired, and that is arranged to repeat the steps of moving and impinging the light beam in an computer-controlled way, in order to supply a predetermined quantity of drug to the skin or tissue 100. The controller 24 preferably has a dosage control feature that registers the effective medicinal area available, so that efficiently all drug substance may be used.

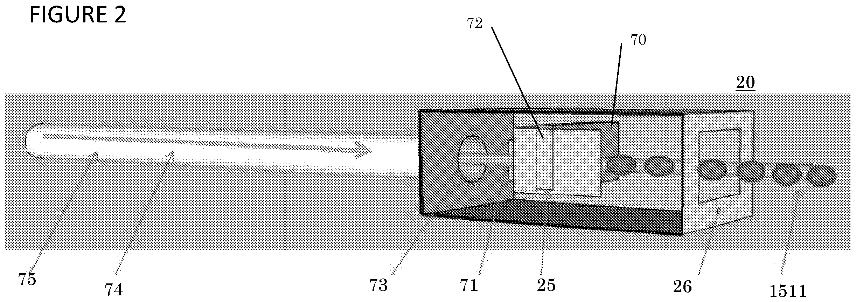

Figure 2 shows a schematic operation of the device of figure 1. Here, light 74 travels in an optical fibre 75 towards handheld delivery device 20, for example, in the form of an endoscope. The device 20 comprises a holder 25 suitable for holding a carrier 70 comprising a drug layer (not shown). The replaceable cartridge 72 can be inserted into the holder 25. A light pointer 26 can be provided to manually control an ejection trajectory of a transferred drug 1511.

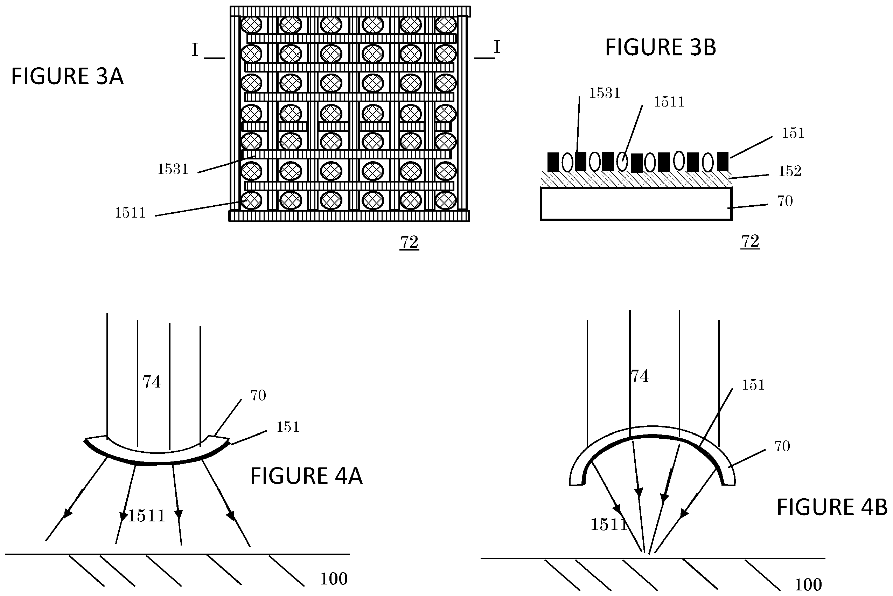

Figure 3A schematically provides an elevational view and side view respectively of a pre-patterned donor cartridge 72 A and B of the donor cartridge, of the type that may be inserted in the holder 25. The drug layer 151 may be pre-patterned, so that enhanced control may be provided on the amount of drug substance 1511 that is ejected. Pre-patterning may be formed by separating the drug substance in sections 1531. The sections may be bounded by reinforcement strips or structures that facilitate transferring a distinct quantity of drug 1511 to the skin or tissue 100.

This is especially useful for viscous types of substances, in a viscosity range of 100-180 Pa.s. Figure 3B shows a side view along the I-I line in Figure 3A of the donor cartridge 72 having three main sections: a transparent carrier 70 such as glass, film or tape; a thin layer of a dynamic release layer 152 and a drug layer 151. The dynamic release layer may be a 10-200 nm triazene polymer layer; and a coated donor material 151. In the example, the donor material 151 is pre-patterned in sections 1531.

Figure 4A and 4B show different side views of the carrier 70, that may be used to direct the drug substance 1511 in a prescribed direction. In the Figure 4A embodiment, the carrier may provided on a rigid substrate 70 having a form design for directing the controlled quantity of drug from the drug layer to a predetermined spot on the skin or tissue 100. The form design may be conical arranged to control an ejection trajectory of a transferred drug. Typically, the ejection trajectory will be normal to the exit surface of the donor material layer 151. Depending on the application, it may be beneficial to focus the ejection trajectory by designing a concave donor layer 151. For instance, this may provide a single spot where the drug substance 1511 may be injected from a variety of ejection trajectories. For surface treatment or in a case a substance 1511 needs to be injected in a wider area, a convex donor layer design can be provided. The carriers 70 be provided by a rotatable plate; wherein the carrier is provided on the plate to move relative to the light beam 74.

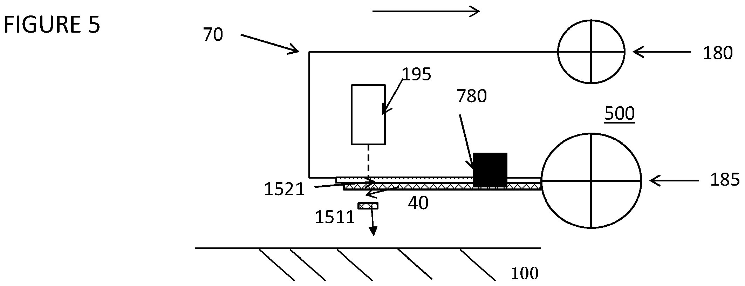

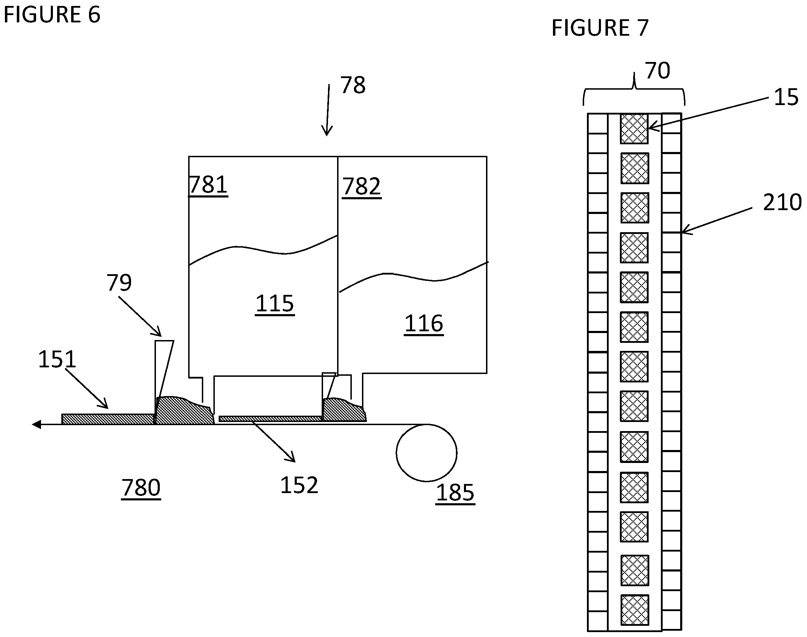

In Figure 5 a cartridge system 500 is disclosed that functions as a carrier transport mechanism arranged to provide a virginal area of the carrier. The cartridge system comprises a reels 185 and 180. By taking up and unwinding said reels, a virginal area of the donor film 70 is exposed to a laser 195 that transfers a drug substance 1511 to the skin or tissue 100. The carrier tape 70 can be made from PET film and maybe passed through a donor feed cartridge system 500 where it is aligned under the beam of a laser 195 and transferred to the skin or tissue 100. The cartridge may be removable from the holder, or the tape system can be integral with it. The donor feed system may comprise a second cartridge 780, combinable with first cartridge system 500, or may be integral with it. The second cartridge 780 may be inserted, e.g. by clicking, combining or fixation, with the first cartridge, to form a replaceable container unit for the drug delivery system 500. In this case, it is advantageous to have the drug substance applied in the form of a flowable drug matter, and a spreader for spreading the drug on the carrier for forming the drug layer. Figure 6 shows a detailed embodiment.

A laser repetition rate may be as high as 60-600kHz. Depending on the laser repetition rate, a donor film refreshment rate may be provided by the carrier transport mechanism at a velocity ranging at from 100micron/s - 0,1 m/s. In order to provide a virginal area a start-stop mechanism may be provided, where a laser beam is scanned over a donor material layer 151 in stop condition, and wherein the material layer is transported in order to provide a next virginal area.

The delivery device may also be moved by a motorized stage (not disclosed) that can be moved in accordance with alignment control means that align the delivery device relative to an injection point.

Figure 6 shows an example of a donor feed system 780, e.g. of the form as disclosed in Figure 5. The donor feed system is provided by a replaceable container 78 containing a flowable drug matter 115, and a spreading mechanism, e.g. in the form of a squeegee 79 for spreading the drug on the carrier for forming a drug layer of substantially uniform height. The layer may be formed by repeated application from different compartments 781, 782, e.g. of a first layer 116 that forms a dynamic release layer 152; and a second layer 151 containing the drug substance. Layers 151 and 152 may be provided on carrier film 70, that is released from reel 185.

Figure 7 shows an example of a structured donor film 70. To enhance the forming of a well-defined bonding matter, the donor film may contain a tape with pre-deposited drug matter i.e. be provided in a premachined form, for example, comprising a sacrificial layer, a pre-patterned conductive die drug layer provided in a matrix 15 of sacrificial material. While the example shows a substrate 70 as a carrier, the drug layer 151, may, depending on the drug substance, be substrate-less, i.e. the drug layer 151 may function as a carrier. A suitable thickness of the homogenous layer may range between 50 and 2000 nm, preferably in a range of 50-500 nm or even more preferably in a range of 50-250 nm. A transport patterning 210 may be provided, for example, in the form of embossing or periodical punching the film, to suitably mark or register the transported film 70.

Other variations to the disclosed embodiments can be understood and by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A device for drug supply, the device comprising:
- a holder suitable for holding a carrier comprising a drug layer (151);
- a transmission part for transmitting a light beam from a light system, via the transmission part to the carrier;
- a placement provision; arranged for distancing the carrier from the skin or tissue; and
- a control mechanism arranged to;
∘ moving the light beam and/or the carrier relative to each other, in order to target a virginal area of the carrier for the light beam;
∘ impinging the light beam via the transmission part on the carrier; in such a way that the carrier is activated to eject a distinct quantity of drug transferred (50) in normal direction from the carrier from the drug layer to the skin or tissue; and
∘ repeating the steps of moving and impinging in a computer-controlled way, in order to supply a predetermined quantity of drug to the skin or tissue.

2. A device according to claim 1, wherein the carrier comprises a dynamic release layer that ejects, under the influence of the light beam a propelling gas for transfer of the drug from the drug layer.

3. A device according to claim 1, wherein the transmission part comprises a beam deflector that is controlled to target said virginal area by deflection of said beam over the drug layer.

4. A device according to claim 1, further comprising a carrier transport mechanism arranged to provide said virginal area of the carrier.

5. A device according to claim 4, wherein the film transport mechanism comprises a cassette insertable in said holder, a take-up reel and an unwind reel provided in said cassette, in order to provide, by taking up and unwinding said carrier, said virginal area of the drug layer.

6. A device according to claim 1-5, wherein the cassette contains a tape with pre-deposited drug matter.

7. A device according to claim 5, wherein the cassette is provided with a replaceable container containing a flowable drug matter, and a spreading mechanism for spreading the drug on the carrier for forming the drug layer.

8. A device according to claim 4 wherein the film transport mechanism is provided by a rotatable plate; wherein the carrier is provided on the plate to move relative to the light beam.

9. A device according to claim 1 wherein the carrier is provided by a rigid substrate (70) having a form design for directing the controlled quantity of drug from the drug layer to a predetermined location on the skin or tissue.

10. A device according to claim 9 wherein the form design is conical arranged to control an ejection trajectory of a transferred drug.

11. A device according to claim 1, wherein the holder comprises a hand grip for hand holding the device; and wherein the holder further comprises a light system.

12. A device according to claim 1, wherein the holder is comprised in an endoscope; and wherein the transmission part comprises an optical fibre.

13. A device according to any of preceding claims, wherein the drug layer has a thickness in a range between 0.5-500 micron.

14. A device according to any of preceding claims, wherein the drug layer is provided with a premachined patterning.

## Patentansprüche

1. Vorrichtung zur Arzneizufuhr, die Vorrichtung umfassend:
- einen Halter, geeignet zum Halten eines Trägers, der eine Arzneischicht (151) umfasst;
- ein Übertragungsteil zum Übertragen eines Lichtstrahls von einem Lichtsystem über das Übertragungsteil an den Träger;
- eine Platzierungseinrichtung; angeordnet zum Beabstanden des Trägers von der Haut oder dem Gewebe; und
- einen Steuermechanismus, angeordnet zum
∘ Bewegen des Lichtstrahls und/oder des Trägers zueinander, um einen unberührten Bereich des Trägers für den Lichtstrahl anzuvisieren;
∘ Aufprallen des Lichtstrahls über das Übertragungsteil auf den Träger; derart, dass der Träger aktiviert wird, eine eindeutige Menge übertragene Arznei (50) in normaler Richtung von dem Träger von der Arzneischicht auf die Haut oder das Gewebe auszustoßen; und
∘ Wiederholen der Schritte des Bewegens und Aufprallens auf computergesteuerte Weise, um die Haut oder das Gewebe mit einer vorbestimmten Menge Arznei zu versorgen.

2. Vorrichtung nach Anspruch 1, wobei der Träger eine dynamische Freisetzungsschicht umfasst, die unter dem Einfluss des Lichtstrahls ein treibendes Gas zur Übertragung der Arznei von der Arzneischicht ausstößt.

3. Vorrichtung nach Anspruch 1, wobei das Übertragungsteil einen Strahlablenker umfasst, der gesteuert wird, um den unberührten Bereich durch Ablenken des Strahls über die Arzneischicht anzuvisieren.

4. Vorrichtung nach Anspruch 1, ferner umfassend einen Trägertransportmechanismus, angeordnet zum Bereitstellen des unberührten Bereichs des Trägers.

5. Vorrichtung nach Anspruch 4, wobei der Filmtransportmechanismus eine in den Halter einsetzbare Kassette, eine Aufwickelspule und eine Abwickelspule, bereitgestellt in der Kassette, umfasst, um durch Aufwickeln und Abwickeln des Trägers den unberührten Bereich der Arzneischicht bereitzustellen.

6. Vorrichtung nach Anspruch 1-5, wobei die Kassette ein Band mit vorher aufgetragener Arzneimasse enthält.

7. Vorrichtung nach Anspruch 5, wobei die Kassette mit einem austauschbaren Behälter, der eine fließfähige Masse enthält, und einen Verteilmechanismus zum Verteilen der Arznei auf dem Träger umfasst, um die Arzneischicht zu bilden.

8. Vorrichtung nach Anspruch 4, wobei der Filmtransportmechanismus von einer drehbaren Platte bereitgestellt ist; wobei der Träger auf der Platte bereitgestellt ist, um sich in Bezug auf den Lichtstrahl zu bewegen.

9. Vorrichtung nach Anspruch 1, wobei der Träger von einem starren Substrat (70) bereitgestellt wird, das ein Formdesign aufweist, um die gesteuerte Menge Arznei von der Arzneischicht zu einer vorbestimmten Stelle auf der Haut oder dem Gewebe zu leiten.

10. Vorrichtung nach Anspruch 9, wobei das Formdesign konisch ist, angeordnet zum Steuern einer Ausstoßbahn einer übertragenen Arznei.

11. Vorrichtung nach Anspruch 1, wobei der Halter einen Handgriff zum Halten der Vorrichtung in der Hand umfasst; und wobei der Halter ferner ein Lichtsystem umfasst.

12. Vorrichtung nach Anspruch 1, wobei der Halter in einem Endoskop enthalten ist; und wobei das Übertragungsteil eine Faseroptik umfasst.

13. Vorrichtung nach einem der vorhergehenden Ansprüche, wobei die Arzneischicht eine Dicke in einem Bereich zwischen 0,5-500 Mikron hat.

14. Vorrichtung nach einem der vorhergehenden Ansprüche, wobei die Arzneischicht mit einer vorbearbeiteten Musterung versehen ist.

## Revendications

1. Dispositif d'administration de médicament, le dispositif comprenant :
- un dispositif de maintien adapté pour maintenir un support comprenant une couche de médicament (151) ;
- une partie de transmission pour transmettre un faisceau lumineux à partir d'un système d'éclairage, via la partie de transmission, vers le support ;
- un élément de placement ; disposé pour maintenir à distance le support de la peau ou du tissu ; et
- un mécanisme de commande conçu pour ;
∘ déplacer le faisceau lumineux et/ou le support l'un par rapport à l'autre, afin de cibler une zone vierge du support pour le faisceau lumineux ;
∘ projeter le faisceau lumineux via la partie de transmission sur le support ; de telle sorte que le support est activé pour éjecter une quantité distincte de médicament transféré (50) dans une direction normale du support depuis la couche de médicament dans la peau ou le tissu ; et
∘ répéter les étapes de déplacement et de projection de manière commandée par ordinateur, afin d'administrer une quantité prédéterminée de médicament dans la peau ou le tissu.

2. Dispositif selon la revendication 1, dans lequel le support comprend une couche de libération dynamique qui éjecte, sous l'influence du faisceau lumineux, un gaz propulsif pour le transfert du médicament depuis la couche de médicament.

3. Dispositif selon la revendication 1, dans lequel la partie de transmission comprend un déflecteur de faisceau qui est commandé pour cibler ladite zone vierge par déviation dudit faisceau sur la couche de médicament.

4. Dispositif selon la revendication 1, comprenant en outre un mécanisme de transport de support agencé pour fournir ladite zone vierge du support.

5. Dispositif selon la revendication 4, dans lequel le mécanisme de transport de film comprend une cassette insérable dans ledit dispositif de maintien, une bobine d'enroulement et une bobine de déroulement agencées dans ladite cassette, afin de fournir, en enroulant et en déroulant ledit support, ladite zone vierge de la couche de médicament.

6. Dispositif selon les revendications 1 à 5, dans lequel la cassette contient un ruban avec une matière médicamenteuse pré-déposée.

7. Dispositif selon la revendication 5, dans lequel la cassette est munie d'un conteneur remplaçable contenant une matière médicamenteuse fluide, et d'un mécanisme d'étalement pour étaler le médicament sur le support afin de former la couche de médicament.

8. Dispositif selon la revendication 4, dans lequel le mécanisme de transport de film est fourni par une plaque rotative ; dans lequel le support est agencé sur la plaque pour se déplacer par rapport au faisceau lumineux.

9. Dispositif selon la revendication 1, dans lequel le support est fourni par un substrat rigide (70) ayant une conception de forme pour diriger la quantité commandée de médicament depuis la couche de médicament jusqu'à un emplacement prédéterminé sur la peau ou le tissu.

10. Dispositif selon la revendication 9, dans lequel la conception de forme est conique et agencée pour commander une trajectoire d'éjection d'un médicament transféré.

11. Dispositif selon la revendication 1, dans lequel le dispositif de maintien comprend une poignée pour tenir le dispositif ; et dans lequel le dispositif de maintien comprend en outre un système d'éclairage.

12. Dispositif selon la revendication 1, dans lequel le dispositif de maintien est inclus dans un endoscope ; et dans lequel la partie de transmission comprend une fibre optique.

13. Dispositif selon l'une quelconque des revendications précédentes, dans lequel la couche de médicament a une épaisseur dans une plage comprise entre 0,5 et 500 microns.

14. Dispositif selon l'une quelconque des revendications précédentes, dans lequel la couche de médicament est munie d'un motif pré-usiné.
